# EUROPÄISCHE PATENTSCHRIFT

(11) **EP 3 140 557 B1**
(45) Veröffentlichungstag und Bekanntmachung des Hinweises auf die Patenterteilung: **02.10.2019**
(21) Anmeldenummer: 15723465.9
(22) Anmeldetag: 08.05.2015
(51) Int. Cl.: F16C 11/12, A61F 2/64, A61F 2/66

(54) **FESTKÖRPERGELENK**
SOLID-BODY JOINT
ARTICULATION À CORPS SOLIDES

(30) Priorität: 08.05.2014 DE 102014006727
(43) Veröffentlichungstag der Anmeldung: 15.03.2017
(73) Patentinhaber: Fraunhofer Gesellschaft zur Förderung der angewandten Forschung E.V., 80686 München (DE)
(72) Erfinder: BREUNINGER, Jannis, 70188 Stuttgart (DE); STARKER, Felix, 70563 Stuttgart (DE); GREUTER, Jonas, 78467 Konstanz (DE)
(74) Vertreter: Rösler, Uwe
(86) Internationale Anmeldenummer: PCT/EP2015/060132
(87) Internationale Veröffentlichungsnummer: WO 2015/169934

(56) Entgegenhaltungen:
- DE-A1- 3 241 373
- DE-A1- 10 330 947
- DE-A1-102007 009 605
- US-A- 3 142 888
- US-A- 4 405 184
- US-A- 5 061 107
- US-A1- 2013 308 997

## Beschreibung

### Technisches Gebiet

Die Erfindung bezieht sich auf ein Festkörpergelenk mit zwei schwenkbar um eine Drehachse angeordneten Lagerelementen sowie wenigstens zwei sich quer zur Drehachse erstreckenden Federelementen, die jeweils einseitig mit einem Lagerelement und andererseits mit dem anderen Lagerelement in Wirkverbindung stehen.

### Stand der Technik

Auf dem Gebiet der mechanischen Konstruktion existiert eine Vielzahl unterschiedlich ausgebildeter Gelenk- und Federtypen, über die wenigstens zwei mechanische Komponenten beweglich, insbesondere drehbeweglich, miteinander verbindbar sind. Technische Gelenke, wie Dreh-, Schraub-, Kugel- oder Kreuzgelenke etc., bestehen aus wenigstens zwei separat gefertigten Bauteilen, die Reibkraft-beaufschlagt miteinander in Wirkverbindung treten und somit stets einem mehr oder minder stark ausgeprägten Verschleiß unterworfen sind, der durch geeignete Schmier- oder Gleitmittel um ein bestimmtes Maß herabgesetzt werden kann.

Gilt es, reibungsbedingte Verluste innerhalb technischer Gelenkkonstruktionen auszuschließen, so eignen sich hierfür so genannte Festkörpergelenke, die eine Relativbewegung zwischen zwei Starrkörperbereichen durch Biegung eines beider Starrkörperbereiche verbindenden Bereiches verminderter Biegefestigkeit ermöglichen. Insbesondere bei aus Kunststoff gefertigten Gelenkkonstruktionen werden so genannte Filmscharniere eingesetzt, die im Wesentlichen aus einer dünnwandigen Verbindung bestehen, die aufgrund ihrer Biegsamkeit eine begrenzte Dreh- oder Schwenkbewegung zwischen zwei einstückig miteinander verbundenen Teilen ermöglicht. Neben der eingeschränkten Beweglichkeit einer derartigen Gelenkverbindung können belastungs- und materialbedingte Ermüdungsbrüche auftreten, die das Gelenk irreversibel schädigen.

Unter der Vielzahl bekannter Ausbildungsformen zur Realisierung eines Festkörpergelenkes sei auf das Prinzip sowie die konstruktive Auslegung des so genannten Kreuzfedergelenkes verwiesen. Ein derartiges Gelenk ist in der Druckschrift US 3,142,888 beschrieben und weist zwei koaxial angeordnete und bereichsweise ineinander greifende Hohlzylinderformabschnitte auf, die ausschließlich über quer zur gemeinsamen Zylinderachse verlaufende Blattfederelemente miteinander verbunden sind. Die blattfederartig ausgebildeten Federelemente, von denen vorzugsweise wenigstens zwei in Kreuzstellung quer zur gemeinsamen Zylinderachse angeordnet sind, ermöglichen ein Verdrehen der beiden ansonsten lose relativ zueinander konzentrisch angeordneten zylinderförmigen Formabschnitte um die als Drehachse ausgebildete gemeinsame Zylinderachse relativ zueinander. Die Blattfederelemente sind dabei jeweils mit beiden Blattfederenden an die Innenwände beider zylinderförmig ausgebildeter Formabschnitte gefügt. Gattungsgemäße Kreuzfedergelenke stellen weitgehend verschleißfreie Festkörpergelenke dar, die zumeist im Messgerätebau als spiel-, reibungs- und abnutzungsfreie Drehgelenke eingesetzt werden. Ergänzend sei in diesem Zusammenhang auch auf die Druckschriften DE 818 293 A sowie DE 1654489 U verwiesen.

Aus der Druckschrift DE 103 30 947 A1 ist ein Kreuzfederelement zu entnehmen, dessen mechanische Belastbarkeit optimiert ist, indem zur Verbindung zweier gegeneinander verdrehbarer, zylinderartig ausgebildeter Lagerelemente, die sich kreuzenden Blattfederelemente mindestens paarweise ausgebildet sind, wobei die Enden jedes Blattfederpaares einer Seite jeweils an unterschiedlichen Lagerelementen befestigt sind.

Neben den aus unterschiedlichen Bauteilen zusammengesetzten Festkörpergelenken, beispielsweise nach Art eines vorstehend erläuterten Kreuzfedergelenkes, sind auch monolithisch gefertigte Festkörpergelenke bekannt, zu deren Erläuterung stellvertretend auf die Druckschrift US 2013/0308997 A1 verwiesen wird, in der eine einstückig gefertigte Gelenkstruktur mit zwei plattenförmig ausgebildeten Lagerelementen beschrieben ist, die zwei einander zugewandte Flächenseiten besitzen, zwischen denen zwei die beiden plattenförmigen Lagerelemente jeweils einstückig miteinander verbindende, in Kreuzstellung zueinander angeordnete Blattfederelemente angeordnet sind. Über die elastisch verformbaren Blattfederelemente lassen sich beide Lagerelemente relativ zueinander um eine durch die Kreuzstellung beider Blattfederelemente vorgegebene Dreh- bzw. Schwenkachse auslenken. Zudem sind beide Lagerelemente aufgrund der elastischen Eigenverformbarkeit der Blattfederelemente federnd zueinander gelagert.

Die Schrift US 4 405 184 A offenbart die Merkmale des Oberbegriffs von Anspruch 1.

### Darstellung der Erfindung

Der Erfindung liegt die Aufgabe zugrunde, ein Festkörpergelenk mit zwei schwenkbar um eine Drehachse angeordneten Lagerelementen sowie wenigstens zwei sich quer zur Drehachse des Festkörpergelenkes erstreckenden Federelementen, die jeweils einseitig mit einem Lagerelement und andererseits mit dem anderen Lagerelement in Wirkverbindung stehen, derart weiterzubilden, dass die durch die Dreh- bzw. Schwenkbewegungen innerhalb des Festkörpergelenkes auftretenden Materialspannungen reduziert und die damit verbundene Belastbarkeit signifikant erhöht werden kann. Darüber hinaus gilt es, ein Federgelenk anzugeben, das zusätzlich zu wenigstens einem rotatorischen federelastischen Freiheitsgrad einen orthogonal zur Drehachse orientierten gleichermaßen federelastischen weiteren Freiheitsgrad besitzt.

Die Lösung der der Erfindung zugrunde liegenden Aufgabe ist im Anspruch 1 angegeben. Den Erfindungsgedanken in vorteilhafter Weise weiterbildende Merkmale sind in den Unteransprüchen sowie der weiteren Beschreibung, insbesondere unter Bezugnahme auf die Ausführungsbeispiele, angegeben.

Lösungsgemäß zeichnet sich ein Festkörpergelenk nach den Merkmalen des Oberbegriffes des Anspruches 1 dadurch aus, dass zwischen beiden Lagerelementen ein spiral- oder helikalförmiges Federelement angeordnet ist, wobei die wenigstens zwei sich quer zur Drehachse erstreckenden Federelemente jeweils einseitig mit dem spiral- oder helikalförmigen Federelement und andererseits jeweils mit einem der beiden Lagerelementen verbunden sind.

Sorgen einerseits die zwischen beiden Lagerelementen sich quer zur Drehachse erstreckenden Federelemente für eine relative Drehbeweglichkeit beider Lagerelemente um die Drehachse, so sorgt andererseits das zwischen beiden Lagerelementen lösungsgemäße angeordnete und ausgebildete spiral- oder helikalförmige Federelement um eine zusätzliche quer zur Drehachse orientierte federelastische Lagerung beider Lagerelemente. Insbesondere verfügt das spiral- oder helikalförmige Federelement zusätzlich zu den sich quer zur Drehachse erstreckenden Federelementen über eine das gesamte Festkörperelement stabilisierende mechanische Abstützfunktion, durch die, ohne die Drehbeweglichkeit des Drehgelenkes nachhaltig zu beeinflussen, eine signifikante Reduzierung der innerhalb der einzelnen Komponenten bzw. Materialabschnitte des Festkörpergelenkes vorherrschenden Materialspannungen erreicht werden kann. Auf diese Weise lässt sich die Belastbarkeit und somit auch die Lebensdauer des Festkörpergelenkes erheblich verbessern.

Unter dem Begriff "spiral- oder helikalförmiges Federelement" ist ein Federelement zu verstehen, dem eine durch seine Form vorgegebene Federachse zuordenbar ist, die durch die Anbringung des Federelementes zwischen beiden Lagerelementen mit der Drehachse des Festkörpergelenkes zusammenfällt oder zumindest zu dieser parallel verläuft. In bevorzugter Weise ist das Federelement helikalförmig ausgebildet und weist zumindest abschnittsweise eine Federgeometrie mit konstanter oder variabler Steigung auf, die sich längs eines Mantels eines virtuellen Geradzylinders windet. Alternativ und in Abhängigkeit zur Ausbildung der Lagerelemente ist eine spiralförmige Federform denkbar, die sich zumindest abschnittsweise mit konstanter oder variabler Steigung längs der Oberfläche eines virtuellen Kegels oder Kegelstumpfes windet. Sämtlichen "spiral- oder helikalförmigen Federelementen" im lösungsgemäßen Sinne ist gemein, dass sie sowohl eine um die Federachse bzw. Drehachse des Festkörpergelenkes orientierte als auch eine quer zur Feder- bzw. Drehachse des Festkörpergelenkes orientierte federelastische Eigenverformbarkeit besitzen, um Lastmomente, die längs eines Kraftflusses zwischen beiden Lagerelementen des Festkörpergelenkes wirken, federnd abzufangen.

Das spiral- oder helikalförmige Federelement ist vorzugsweise in Form eines zu einer Spirale oder Helix geformten Federbandes mit einem rechteckförmigen Federquerschnitt ausgebildet, dessen zur Federachse radiale Außenseite den Lagerelemeten zugewandt ist und an dessen Innenseite jeweils einseitig die Enden der sich quer zur Drehachse des Festkörpergelenkes erstreckenden Federelemente lokal verbunden sind. Die Ausbildung und Anordnung des spiral- oder helikalförmigen Federelementes zwischen beiden Lagerelementen ist insbesondere durch die lose Lagerung des Federelementes zwischen beiden Lagerelementen charakterisiert. Die gegenüber den Lagerelementen lose Lagerung des spiral- oder helikalförmigen Federelementes wird durch die sich quer zur Drehachse erstreckenden Federelementen ermöglicht, durch deren Federlänge und Anordnung der gegenseitige Abstand beider Lagerelemente festgelegt ist.

Die vorzugsweise in Form von Blattfedern ausgebildeten, sich quer zur Drehachse erstreckenden Federelemente, deren Blattfederenden einseitig direkt mit einem Lagerelement und andererseits mit dem spiral- oder helikalförmigen Federelement in Wirkverbindung stehen, stellen jeweils zweiseitig fest eingespannte Federbalken dar, die sowohl den Abstand zwischen beiden Lagerelementen als auch die Drehachse des Festkörpergelenkes vorgeben. Die jeweils beidseitig fest eingespannten, sich quer zur Drehachse erstreckenden Blattfederelemente sind in einer Ausführungsvariante jeweils als separate Federelemente ausgebildet, die jeweils axial zur Feder- bzw. Drehachse des Festkörpergelenkes beabstandet zueinander angeordnet sind. In einer einfachsten Ausführungsform sind wenigstens zwei sich quer zur Drehachse erstreckende Federelemente erforderlich, von denen ein erstes Federelement einseitig mit dem einen Lagerelement und andererseits mit dem spiral- oder helikalförmigen Federelement und ein zweites Federelement einseitig mit dem anderen Lagerelement und ebenfalls mit dem spiral- oder helikalförmigen Federelement verbunden sind. Auf diese Weise tritt das spiral- oder helikalförmige Federelement zwar nicht in unmittelbaren Kontakt zu beiden Lagerelementen und behält sich somit die dem Federelement zu eigenen Federeigenschaften unverändert bei, dient jedoch zugleich mittelbar als kraftübertragendes Abstützelement für Lasten, die längs eines zwischen beiden Lagerelementen verlaufenden Kraftflusses über die sich quer zur Drehachse erstreckenden Federelemente wirken.

In einer weiteren, bevorzugten Ausführungsform des lösungsgemäß ausgebildeten Festkörpergelenkes nach Art eines Kreuzfedergelenkes befinden sich die wenigstens zwei quer zur Drehachse erstreckenden Federelemente in Kreuzstellung, d.h. in Projektion längs der Drehachse des Festkörpergelenkes schließen die Längserstreckungen der im Lastfreien Zustand gerade ausgebildeten Blattfederelemente vorzugsweise einen 90°-Winkel ein. Auch in diesem Fall können die einzelnen sich quer zur Drehachse erstreckenden Federelemente separat zueinander ausgebildet sein, gleichwohl ist es denkbar, axial längs zur Drehachse des Festkörpergelenkes angeordnete, sich quer zur Drehachse erstreckende Federelemente im Bereich der Kreuzungspunkte miteinander zu verbinden, um auf diese Weise die Drehachse des Festkörpergelenkes räumlich und körperlich festzulegen. Konstruktionsbedingt ist die maximale Schwenkbewegung eines derartig ausgebildeten Festkörpergelenkes auf einen maximalen Drehwinkel von 90° beschränkt. Gilt es, den Drehwinkel des Festkörpergelenkes zu vergrößern, so sieht eine weitere Ausführungsform für ein lösungsgemäß ausgebildetes Festkörpergelenk eine Vorkrümmung der sich quer zur Drehachse erstreckenden Federelemente in einem unbelasteten, d.h. nicht ausgelenkten Zustand des Festkörpergelenkes derart vor, dass bei Auslenkung des Festkörpergelenkes die Krümmung der einzelnen Federelemente reduziert wird. Wie die weitere Beschreibung, insbesondere unter Bezugnahme auf ein diesbezügliches illustriertes Ausführungsbeispiel zeigen wird, sind mit der vorstehenden Maßnahme Drehbewegungen von 120° und mehr möglich.

Eine weitere Ausführungsform des lösungsgemäß ausgebildeten Festkörpergelenkes sieht eine zusätzliche Maßnahme vor, durch die die Drehbeweglichkeit des Festkörpergelenkes in Abhängigkeit der auf das Festkörpergelenk einwirkenden Last eingeschränkt bzw. blockiert werden kann. Eine derartige Blockierung bzw. Sperrung der Gelenkfunktion kann insbesondere in Anwendung und Ausbildung des lösungsgemäßen Festkörpergelenkes in Form eines Prothesen-Kniegelenkes von Vorteil sein. Zur Blockade der Gelenkfunktion sieht das Festkörpergelenk an dem spiral- oder helikalförmigen Federelement und/oder an den Lagerelementen einander zugewandte Strukturen vor, die bei gegenseitiger Annäherung ineinandergreifen und ein Schwenken beider Lagerelemente um die Drehachse verhindern. Derartig einander zugewandte Strukturen können in vorteilhafter Weise in Form von Verzahnungen ausgebildet sein. Weitere Einzelheiten hierzu sowie Möglichkeiten einer entsprechenden diesbezüglichen Realisierung sind im Rahmen der nachstehenden Illustrationen näher erläutert.

Durch die kompakte Bauform des lösungsgemäßen Festkörpergelenkes, das in besonders vorteilhafter Weise monolithisch ausgebildet ist, d.h. beide Lagerelemente sowie die dazwischen befindlichen, sich quer zur Drehachse erstreckenden Federelemente sowie das spiral- oder helikalförmige Federelement sind einstückig gefertigt, vorzugsweise im Rahmen eines generativen Fertigungsprozesses hergestellt, beispielsweise im Rahmen des selektiven Lasersinterns, eröffnet sich die konstruktive Möglichkeit, dass wenigstens zwei Festkörpergelenke in serieller oder paralleler mechanischer Wirkweise miteinander verkoppelt werden können. Im Falle der seriellen Kopplung ist jeweils ein Lagerelement eines ersten Festkörpergelenkes fest mit dem anderen Lagerelement eines zweiten Festkörpergelenkes verbunden, so dass die Drehachsen beider Festkörpergelenke koaxial zueinander gelagert sind, d.h. zusammenfallen. Im Falle der parallelen mechanischen Kopplung sind die Lagerelemente wenigstens zweier Festkörpergelenke derart miteinander verbunden, so dass die Drehachsen beider Festkörpergelenke parallel und voneinander beabstandet gelagert sind.

Das lösungsgemäß ausgebildete Festkörpergelenk, das neben seiner Drehbeweglichkeit über federnd gelagerte Eigenschaften verfügt, eignet sich in besonders vorteilhafter Weise für den Einsatz in der Medizintechnik. Künstliche Gelenke für Exo- und Endo-Prothesen sowie auch Orthesengelenke können mit Hilfe des lösungsgemäßen Festkörpergelenkes einem breiten Einsatzspektrum zugeführt werden, zumal insbesondere die generative Herstellungstechnik eine große Variantenvielfalt in Bezug auf Skalierung und konstruktive Komplexität im Gelenkaufbau ermöglicht. So können die Federeigenschaften sowie auch der Schwenkbereich, um die die Lagerelemente relativ zueinander um eine dem Festkörpergelenk zugeordnete Drehachse geschwenkt werden können, unabhängig voneinander variabel eingestellt und aufeinander abgestimmt werden.

Die Variabilität in Hinblick auf die elastische Eigenverformbarkeit des Festkörpergelenkes sowie auch des Schwenkbereiches lässt sich zudem durch den Einsatz verschiedenartiger Materialien, die über unterschiedliche elastische Eigenschaften verfügen, erheblich vergrößern. Insbesondere der Einsatz moderner generativer Herstellungsverfahren ermöglicht die Verarbeitung unterschiedlicher Materialien. So ließen sich spiral- oder helikalförmige Federelemente fertigen, die abschnittsweise unterschiedliche elastische Eigenschaften aufweisen, wodurch das elastische Eigenverformungsverhalten individuell eingestellt und vorgegeben werden kann, um beispielsweise das Schwenkverhalten des Festkörpergelenkes, bspw. in Bezug auf einen winkelabhängigen variablen Kraftaufwand für ein Schwenken und /oder in Bezug auf eine mögliche Einflussnahme auf die räumliche Lage der Drehachse während des Schwenkvorganges, die sich bspw. längs einer Polkurve bewegt, gezielt zu beeinflussen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a, b: perspektivische Ansicht sowie axiale Frontansicht eines lösungsgemäß ausgebildeten Festkörpergelenkes,
- Fig. 2, 3: alternative Ausführungsformen für ein lösungsgemäßes Festkörpergelenk,
- Fig. 4a, b: perspektivische Ansicht sowie axiale Frontansicht an ein Festkörpergelenk mit vergrößertem Schwenkbereich,
- Fig. 5: Darstellungen zur Illustration alternativ ausgebildeter spiral- oder helikalförmiger Federelemente,
- Fig. 6: perspektivische Ansicht eines Ausführungsbeispiels für ein lösungsgemäßes Festkörpergelenk,
- Fig. 7, 8: parallele sowie serielle mechanische Verschaltung wenigstens zweier Festkörpergelenke,
- Fig. 9 a, b: Festkörpergelenk mit Blockierverzahnung gegen Schwenken,
- Fig. 10 a, b: alternative Ausführungsform für ein lösungsgemäßes Festkörpergelenk,
- Fig. 11 a-d: bevorzugte Verwendungsbeispiele für ein Festkörpergelenk sowie
- Fig. 12: alternative Ausführungsform für ein spiral- oder helikalförmiges Federelement.

### Wege zur Ausführung der Erfindung, gewerbliche Verwendbarkeit

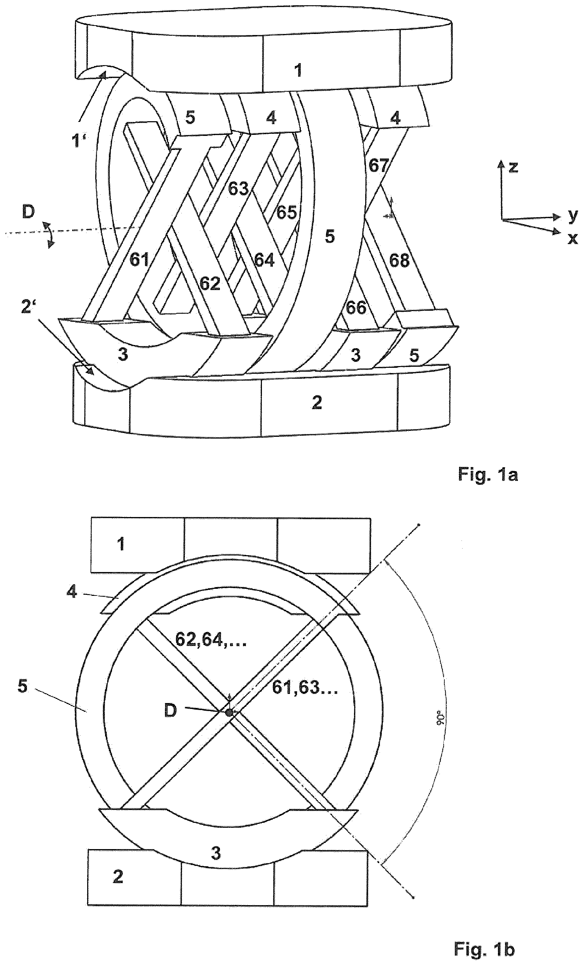

Fig. 1a zeigt in perspektivischer Darstellung eine Ausführungsform eines lösungsgemäß ausgebildeten Festkörpergelenkes mit zwei im Wesentlichen plattenförmig ausgebildeten Lagerelementen, einem oberen Lagerelement 1 und einem unteren Lagerelement 2, die jeweils einander zugewandte Lagerflächen 1', 2' aufweisen. Die Lagerelemente 1, 2 können je nach Einsatzzweck individuell ausgestaltet sein und sind nicht auf die dargestellte Plattenform festgelegt. Einstückig mit den Lagerelementen 1, 2 verbunden erheben sich über die jeweiligen Lagerflächen 1', 2' bogensegmentartig ausgebildete Abstützelemente 3, 4, die über dem jeweils gegenüber liegenden Lagerelement 1, 2 zugewandt orientierte Abstützflächen 3', 4' verfügen. In dem in Figur 1a dargestellten Ausführungsbeispiel weisen die Lagerelemente 1, 2 jeweils zwei in y-Richtung - siehe das in Figur 1a dargestellte kartesische Koordinatensystem - voneinander beabstandete, ansonsten gleichförmig ausgebildete Abstützelemente 3, 4 auf. Die an den jeweils gegenüber liegenden Lagerflächen 1', 2' angebrachten Abstützelemente 3, 4 sind in Bezug auf ihre gegenseitige räumliche Anordnung längs der y-Achse auf Lücke angeordnet, so dass ein spiral- oder helikalförmiges Federelement 5, in Form und Größe angepasst, zwischen den Abstützelementen 3, 4 kontaktfrei eingebracht ist. Das spiral- oder helikalförmige Federelement 5 ist ansonsten gegenüber den Lagerflächen 1', 2' des jeweils oberen und unteren Lagerelements 1, 2 lose, d.h. mit Abstand, gelagert.

Die körperliche Verbindung zwischen dem oberen Lagerelement 2, dem unteren Lagerelement 2 sowie dem zwischen beiden Lagerelementen 1, 2 eingebrachten spiral- oder helikalförmigen Federelement 5 erfolgt über sich quer zur y-Raumachse erstreckenden blattfederförmig ausgebildeten Federelemente 61 bis 68. Die blattfederartig ausgebildeten Federelemente 61 bis 68 sind jeweils einseitig mit dem spiral- oder helikalförmigen Federelement 5 und andererseits jeweils mit einem der beiden Lagerelemente 1, 2 verbunden, vorzugsweise monolithisch verbunden. Dabei stützt sich beispielsweise das Federelement 61 mit seinem unteren Ende unmittelbar an der Abstützfläche 3' des Abstützelementes 3 ab, wohingegen das obere Ende des Federelementes 61 unmittelbar mit dem spiral- oder helikalförmigen Federelement 5 verbunden ist. In gleicher Weise ist das in y-Richtung nächstfolgende Federelement 62 verbunden. Hingegen stützt sich das blattfederartig ausgebildete Federelement 63 mit seinem unteren Ende unmittelbar an dem spiral- oder helikalförmigen Federelement 5 ab, wohingegen das obere Ende des Federelementes 63 mit dem oberen Abschnittselement 4 monolithisch verbunden ist. In gleicher Weise stützt sich das in y-Richtung nächstfolgende Federelement 64 mit seinem oberen, nicht dargestellten Federende am oberen Abstützelement 4 ab und mit seinem unteren Federende am spiral- oder helikalförmigen Federelement 5. In entsprechender Weise erfolgt die Abstützung bzw. Verbindung der in y-Richtung folgenden blattförmigen Federelemente 66 bis 68. Die Längen der einzelnen Federelemente 61 bis 68, deren räumliche Anbringung sowie der Durchmesser des spiral- oder helikalförmigen Federelementes 5 sind derart aufeinander abgestimmt, dass zwischen dem Federelement 5 und dem oberen bzw. unteren Lagerelement 1, 2, jeweils ein lichter Abstand ist.

Das in Figur 1a dargestellte Festkörpergelenk, das in vorteilhafter Weise monolithisch, d.h. einstückig gefertigt ist, vorzugsweise unter Einsatz eines generativen Herstellungsverfahrens, verfügt konstruktionsbedingt über eine Drehachse D, die durch die Kreuzstellung der blattfederartig ausgebildeten Federelemente 61 bis 68 räumlich bestimmt ist.

Durch die gegenüber dem oberen und unteren Lagerelement 1, 2 lose Lagerung des spiral- oder helikalförmigen Federelementes 5, das ausschließlich jeweils mit den einen Enden der blattfederartig ausgebildeten Federelemente 61 bis 68 monolithisch verbunden ist, sind die elastischen Eigenschaften des Festkörpergelenkes durch zwei unterschiedliche Federarten bestimmt, nämlich zum einen durch die elastische Eigenverformbarkeit der jeweils zweiseitig eingespannten, blattfederartig ausgebildeten Federelemente 61 bis 68 sowie zum anderen durch die elastische Eigenverformbarkeit des spiral- oder helikalförmigen Federelementes 5.

Insbesondere das spiral- oder helikalförmige Federelement 5 vermag einen entscheidenden Beitrag zu der Drehbeweglichkeit des Festkörpergelenkes um die Drehachse D beizutragen, der durch die bewegungsabhängige Verdrillung bzw. Aufdehnung der Spiral- oder Helikalform des Federelementes 5 eine um die Drehachse D orientierte Kraft zu generieren vermag. Zusätzlich besitzt das spiral- oder helikalförmige Federelement 5 über eine quer zur Drehachse D orientierte elastische Eigenverformbarkeit, durch die das Festkörpergelenk zusätzlich federnde Eigenschaften erhält. Diese zusätzliche federnde Eigenschaft ist insbesondere beim Einsatz des Festkörpergelenkes im Medizinbereich, insbesondere für künstliche Gelenke, von großem Nutzen und Vorteil, wie dies im Weiteren noch näher erläutert wird.

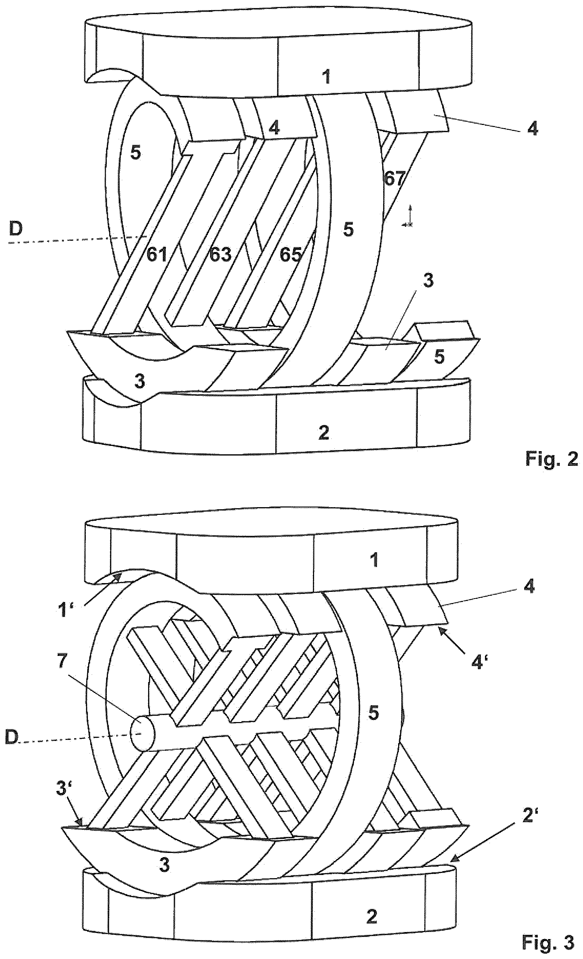

Eine alternative Ausführungsform des in Figur 1a illustrierten Festkörpergelenkes ist in Figur 2 dargestellt, das unter Weglassung der in Figur 1a dargestellten blattfederartig ausgebildeten Federelemente 62, 64, 66 und 68 ansonsten baugleich mit dem in Figur 1a illustrierten Festkörpergelenk ist. So stützt sich das spiral- oder helikalförmige Federelement 5 des in Figur 2 illustrierten Festkörpergelenkes ausschließlich an den vier quer zur y-Achse verlaufenden Federelementen 61, 63, 65 sowie 67 ab, die jeweils einseitig mit einem Abstützelement 3, 4 und andererseits mit dem spiral- oder helikalförmigen Federelement 5 verbunden sind.

Das in Figur 2 illustrierte Festkörperelement verfügt verglichen mit dem in Figur 1a illustrierten Festkörperelement über eine geringere Federsteifigkeit um die Drehachse D des Festkörpergelenkes, die parallel zur angegebenen y-Achse jeweils durch die geometrischen Mitten der Federelemente 61 bis 67 verläuft.

Ein weiteres Ausführungsbeispiel zur Ausgestaltung eines lösungsgemäßen Festkörpergelenkes ist in Figur 3 illustriert. Wieder ausgehend von dem in Figur 1a dargestellten Festkörpergelenk unterscheidet sich das in Figur 3 illustrierte Festkörperelement lediglich dadurch, dass die blattfederartig ausgebildeten Federelemente 61 bis 68 nicht wie im Fall der Figur 1a in y-Richtung separat voneinander ausgebildet sind, sondern längs der Drehachse D durch ein axial verlaufendes Verbindungsmittel 7 allesamt körperlich verbunden sind. Das stabförmig ausgebildete Verbindungsmittel definiert somit körperlich die Drehachse D des Festkörpergelenkes.

Sämtlichen, in den Figuren 1 bis 3 erläuterten Ausführungsbeispielen ist gemeinsam, dass ihr maximaler um die Drehachse D erzielbarer Schwenkbereich auf 90° begrenzt ist, wie dies aus Figur 1b hervorgeht. Dies liegt zum einen an der Ausbildung und Anordnung der Lagerelemente 1, 2 mit dem jeweils einstückig mit diesen verbundenen Abstützelementen 3, 4, die bei einer Maximalverschwenkung um 90° mechanisch jeweils mit dem gegenüber liegenden Lagerelement in Kontakt treten. Zum anderen ist der Gelenkwinkelbereich auf 90° auch durch die nur limitierte elastische Eigenverformbarkeit der blattfederartig ausgebildeten Federelemente 61 bis 68 begrenzt. Aus einer Grundstellung, die in den Figuren 1a, b illustriert ist, in der die einzelnen Blattfedern 61 bis 68 kraftfrei und unverformt, jeweils eine geradlinige Form und in axialer Projektion zur Drehachse D eine 90°-Kreuzstellung einnehmen, werden die Federelemente in eine maximale Endlage geschwenkt, in der die blattfederartigen Federelemente 61 bis 68 halbkreisartig geformt sind.

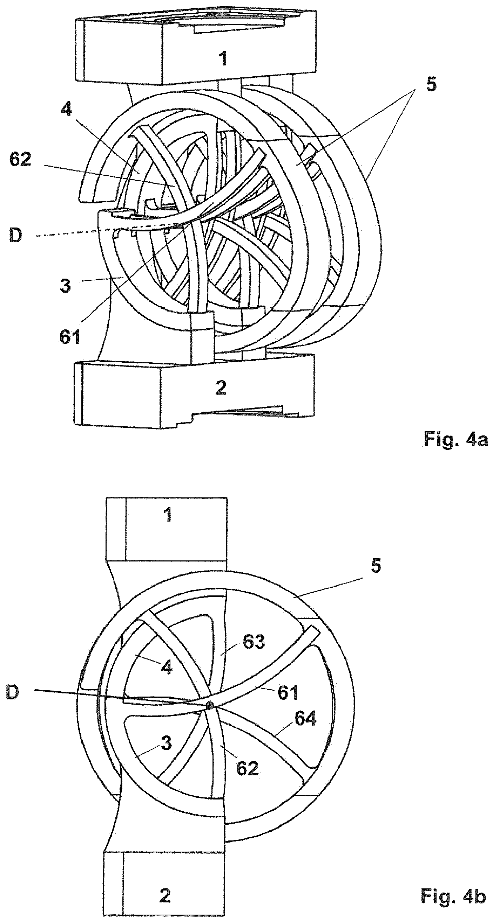

Wird hingegen ein Festkörpergelenk nach lösungsgemäßer Art mit einem größer bemessenen Gelenkwinkelbereich gewünscht, so sei in diesem Zusammenhang auf ein in den Figuren 4a und 4b illustriertes Ausführungsbeispiel verwiesen. Anhand der perspektivischen Ansicht des Festkörpergelenkes gemäß Figur 4a sowie der axialen Draufsicht gemäß Figur 4b ist ersichtlich, dass die blattfederartig ausgebildeten Federelemente 61 bis 68 in der lastfreien Ausgangsstellung eine Vorkrümmung jeweils in Längserstreckung derart aufweisen, dass bei Auslenkung des Festkörpergelenkes die Krümmung des jeweiligen Federelementes 61 bis 68 zunächst reduzierbar ist. Wird das in den Figuren 4a, 4b in seiner Ausgangsstellung gezeigte Festkörpergelenk um die Drehachse D in Uhrzeigerrichtung geschwenkt, so nimmt die Krümmung der einzelnen Federelemente 61 bis 68 zunächst ab bis ein Zustand eines geradlinigen Verlaufes jedes einzelnen Federelementes erreicht wird. Bei weiterem Schwenken des oberen Lagerelements 1 um die Drehachse D nehmen die Federelemente 61 bis 68 eine entgegengesetzt zur Anfangskrümmung orientierte Krümmung an.

Neben der im Ausgangszustand des Festkörpergelenkes vorgesehenen Vorkrümmung längs jedes einzelnen blattfederartig ausgebildeten Federelementes 61 bis 68 sind die Ausgestaltung sowie auch Anbringung der Abstützelemente 3, 4 an den Lagerelementen 1, 2 für einen vergrößerten Schwenkbereich gleichsam von Bedeutung. So sind die bogenförmig ausgebildeten Abstützelemente 3, 4 verglichen zur Ausführungsform gemäß Figur 1a gegenüber der Lagerfläche 1', 2' der Lagerelemente 1, 2 erhöht angebracht. Zudem sind die bogenförmig ausgebildeten Abstützelemente 3, 4 um 45° zur Anordnung gemäß dem Ausführungsbeispiel in Figur 1a um die Drehachse D versetzt angeordnet, um auf diese Weise ein freies Verschwenken beispielsweise des oberen Lagerelementes 1 um die Drehachse D relativ zum unteren Lagerelement 2 in einem Schwenkbereich von wenigstens 120° zu ermöglichen.

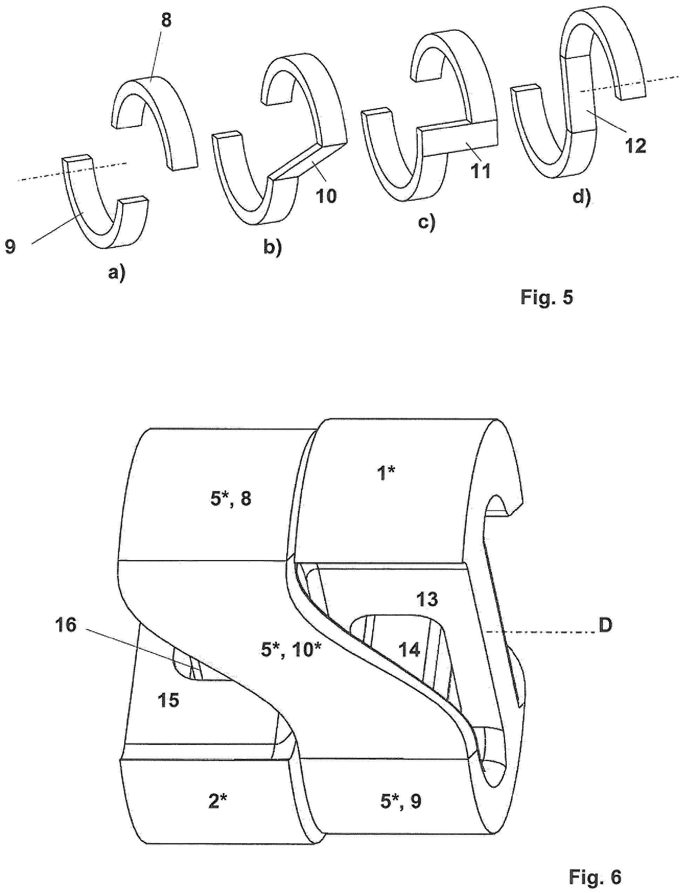

In den Figuren 5a bis d sind alternative Ausführungsformen für eine mögliche Ausgestaltung eines spiral- oder helikalförmigen Federelementes dargestellt. Figur 5a zeigt einen dem oberen Lagerelement zugewandt orientierten ersten Bogenabschnitt 8 sowie einen zweiten, dem unteren Lagerelement zugewandt orientierten Bogenabschnitt 9 des spiral- oder helikalförmigen Federelementes 5, die, wie die weiteren Teilfiguren der Figur 5 zeigen werden, zu Zwecken eines formelastischen Kraftschlusses in unterschiedlicher Weise miteinander verbunden sein können. In Figur 5b dient ein geradlinig trapezförmig ausgebildeter Verbindungsabschnitt 10 zur formelastischen Verbindung beider Bogenabschnitte 8, 9. Figur 5c illustriert eine formelastische Verbindung 11 über einen parallel zur Feder- bzw. Drehachse D des spiral- bzw. helikalförmig ausgebildeten Federelementes. In Figur 5d ist der Verbindungsabschnitt 12 derart ausgebildet, so dass beide Bogenabschnitte 8, 9 in Ergänzung eine klassische Helikalform bilden.

Die in Figur 5a-d illustrierten Möglichkeiten zur Ausbildung eines spiral- oder helikalförmigen Federelementes stellt eine nicht begrenzte Vielfalt an möglichen Ausgestaltungen für ein spiral- oder helikalförmig ausgebildetes Federelement dar.

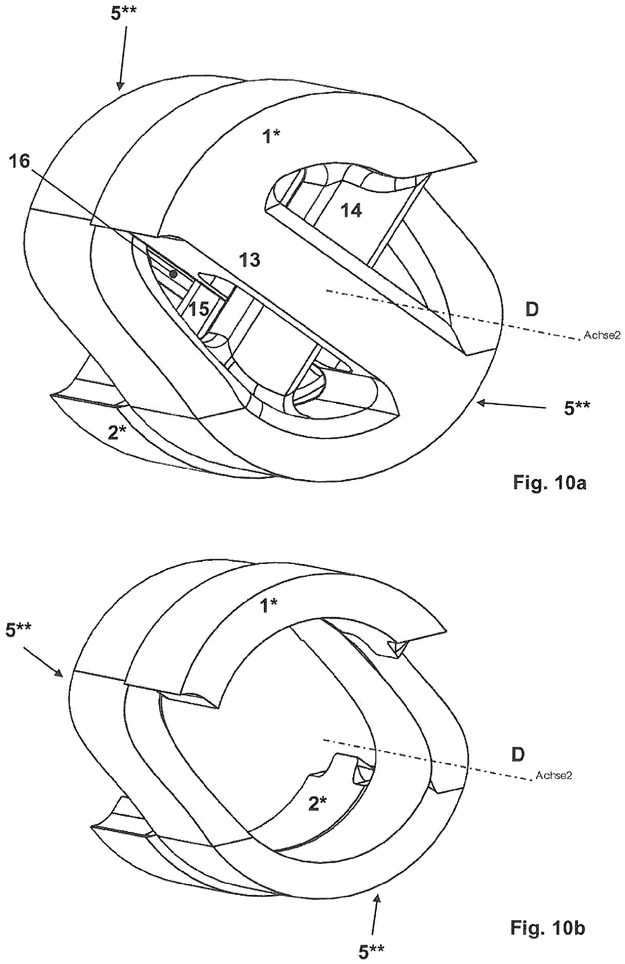

Figur 6 zeigt eine alternative Ausführungsform für ein Festkörpergelenk, das über ein bogenförmig ausgebildetes oberes Lagerelement 1* sowie ein bogenförmig ausgebildetes unteres Lagerelement 2* verfügt. Zwischen beiden Lagerelementen 1*, 2* ist ein helikal geschwungenes Federelement 5* angeordnet, das keinen unmittelbaren körperlichen Kontakt zum oberen und unteren Lagerelement 1*, 2* besitzt. Das obere Lagerelement 1* ist über zwei sich kreuzende, quer zur Drehachse D des Festkörpergelenkes verlaufende Federelemente 13, 14 mit dem helikal geschwungenen Federelement 5* verbunden. Es sei angenommen, dass das Federelement 5* in Art der in Figur 5 b dargestellten Federform ausgebildet ist, d.h. der in der Darstellung obere Bogenabschnitt 8 ist lediglich über den in Figur 6 sichtbaren Verbindungsabschnitt 10* mit dem unteren Bogenabschnitt verbunden. Das mit dem Bezugszeichen 13 versehene Federelement ist an seinem oberen Ende mit dem oberen Lagerelement 1* verbunden und zweigt sich in seiner Längserstreckung gabelförmig auf und ist mit seinem unteren Ende bzw. seinen beiden unteren Enden monolithisch mit dem helikal geschwungenen Federelement 5* verbunden. Der gabelförmige Bereich des Federelementes 13 wird durchragt von dem Federelement 14, das gleichsam das obere Lagerelement 1* mit dem helikal geschwungenen Federelement 5* monolithisch verbindet. In gleicher Weise ist das untere Lagerelement 2* über ein gabelförmig ausgebildetes Federelement 15 sowie ein Federelement 16 mit dem helikal geschwungenen Federelement 5' verbunden. Auch das in Figur 6 illustrierte Festkörpergelenk verfügt um die Drehachse D drehelastische Eigenschaften, gepaart mit quer zur Drehachse D orientierten federelastischen Eigenschaften. Das Federelement 5* stellt eine weitere Raumform dar, die unter dem Begriff des "spiral- oder helikalförmigen Federelementes" zu subsumieren ist. Eine mit dem vorstehenden Festkörpergelenk vergleichbare weitere Ausführungsform ist in Figur 10 gezeigt.

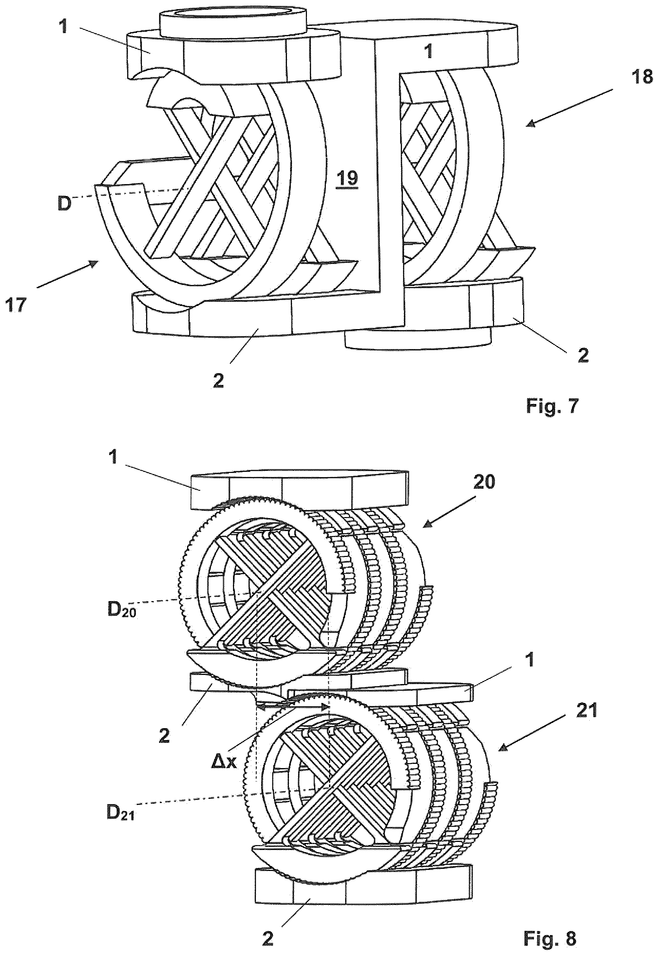

Zur Vergrößerung des Bewegungsspielraumes sowie auch zur Repositionierung der einem Festkörperdrehgelenk zuordenbaren Drehachse können auch mehrere Festkörpergelenke mechanisch parallel oder in Reihe miteinander gekoppelt bzw. geschaltet werden. In Figur 7 ist ein Ausführungsbeispiel, bestehend aus zwei Festkörpergelenken 17, 18, gezeigt, die parallel miteinander verbunden sind, d.h. die Drehachsen D beider Festkörpergelenke 17, 18 sind koaxial, d.h. längs einer gemeinsamen Drehachse angeordnet. Hierbei ist das untere Lagerelement 2 des ersten Festkörpergelenkes 17 über einen Verbindungsabschnitt 19 mit dem oberen Lagerelement 1 des zweiten Festkörpergelenkes 18 verbunden. Eine derartige mechanische Kopplung zweier Festkörpergelenke führt zu einem größeren Bewegungsspielraum des Gesamtgelenkes, jedoch bleibt die zentrale Drehachse D, die sich durch die Drehachsen beider Festkörpergelenke zusammensetzt, erhalten.

In Figur 8 ist ein Ausführungsbeispiel gezeigt, bei dem zwei Festkörpergelenke 20, 21 mechanisch in Reihe geschaltet sind. Hierbei ist das untere Lagerelement 2 des oberen Festkörpergelenkes 20 mit dem oberen Lagerelement 1 des unteren Festkörpergelenkes 21 verbunden. Ist die Verbindung beider Festkörpergelenke 20, 21, wie in Figur 8 gezeigt derart ausgebildet, so dass ihre Drehachsen D in vertikaler Projektion einen Versatz Δx aufweisen, so kann die Drehachse des Gesamtgelenkes gezielt verlagert werden. Durch diese Verlagerung bewegt sich die Drehachse des Gesamtfestkörpergelenkes bei Verdrehen des oberen Lagerelementes 1 gegenüber dem unteren Lagerelement 2 längs einer Pohlkurve.

Eine Verlagerung der Drehachse längs einer Polkurve in Abhängigkeit von der Auslenkung kann auch innerhalb eines einzigen lösungsgemäß ausgebildeten Festkörpergelenkes erzielt werden, nämlich durch geeignete Modifikation des spiral- oder helikalförmigen Federelementes, indem beispielsweise das Federelement abschnittsweise aus unterschiedlichen Federelementmaterialien gefertigt ist, die jeweils über unterschiedliche Federhärten verfügen. Derartige individuelle Ausgestaltungsmöglichkeiten einzelner Komponenten, aus denen das lösungsgemäße Festkörpergelenk zusammengesetzt ist, lassen sich vorzugsweise im Rahmen generativer Herstellungsverfahren, wie beispielsweise Rapid Prototyping oder Additiv Manufacturing realisieren. Insbesondere für das Anwendungsgebiet der Medizintechnik sind individuelle Ausgestaltungsmöglichkeiten von hoher Bedeutung. Die Variationsvielfalt der Gelenke in Verbindung mit der individuellen Fertigungsweise von generativen Verfahren bildet den Grundstein von hoch innovativen Produkten, wie beispielsweise die Anfertigung patientenindividueller Exo- und Endo-Prothesen sowie auch Orthesengelenke.

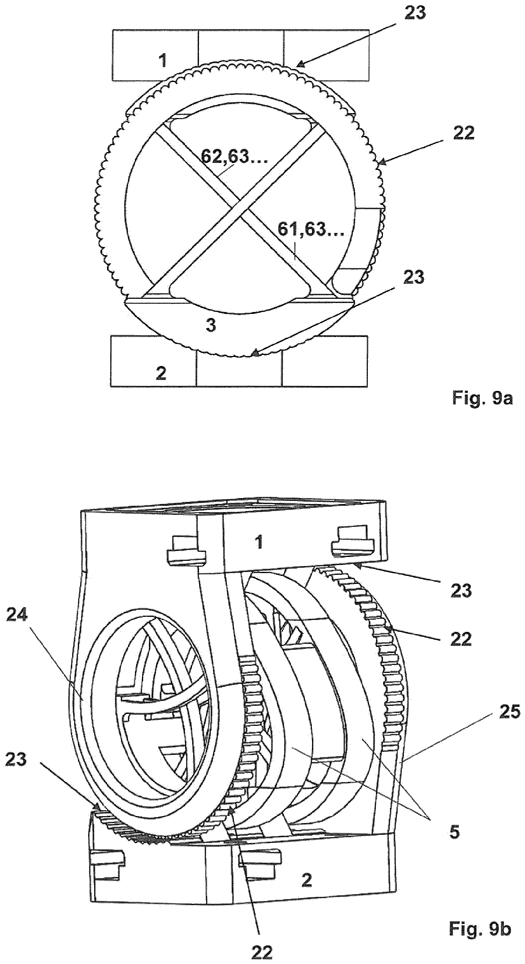

Insbesondere für medizinische Anwendungen, vor allem zur Realisierung künstlicher Gelenke, kann es von Vorteil sein, wenn die Gelenkfunktion beispielsweise bei einer Überbelastung des Gelenkes blockiert wird. Figur 9a stellt eine mögliche Ausführungsform für ein lösungsgemäß ausgebildetes Festkörpergelenk mit Blockierfunktion dar. Hierzu sieht die Außenkontur des spiral- oder helikalförmigen Federelementes 5 eine Außenverzahnung 22 vor. Zusätzlich sehen das obere Lagerelement 1 und/oder das untere Lagerelement 2 jeweils eine dem Federelement 5 zugewandte Verzahnungsstruktur 23 vor. Solange das Festkörpergelenk nicht überlastet ist, sind beide Verzahnungsstrukturen 22, 23 durch einen Spalt getrennt und greifen nicht ineinander. Wird hingegen das Festkörpergelenk bei vertikaler Lasteinwirkung überbelastet, so kommt es zu einer elastischen Eigenverformung sowohl der blattfederartig ausgebildeten Federelemente 61 bis 68 sowie insbesondere des spiral- bzw. helikalförmigen Federelementes 5, wodurch die Verzahnungsstrukturen in gegenseitigen Eingriff gelangen und das Gelenk blockieren. Diese Eigenschaft kann in vorteilhafter Weise bei Prothesen-Kniegelenke genutzt werden. Eine derartige Sperrung bzw. Blockade entsteht unabhängig von der Position bzw. Auslenkung des Festkörpergelenkes, sobald eine quer zur Drehachse orientierte Last eine vorgebbare Lastgrenze überschreitet.

In Figur 9b ist eine alternative Ausbildungsform für eine derartige Gelenkblockade illustriert, bei der fest jeweils mit einem Lagerelement verbundene Strukturen 24, 25 über geeignete Außenverzahnungen 21 verfügen, die bei entsprechender Überlastung des Festkörpergelenkes in entsprechend am gegenüber liegenden Lagerelement 1, 2 angebrachte Gegenstrukturen in Eingriff gelangen. Ansonsten entspricht das Festkörpergelenk in Art und Ausbildung den vorstehend beschriebenen Ausführungsbeispielen.

Figur 10 a zeigt ein weiteres Ausführungsbeispiel für ein Festkörpergelenk, das im Unterschied zu dem in Figur 6 dargestellten Gelenk ein zu einem vollständigen Ringschluss ergänztes helikalförmiges Federelement 5** besitzt. Zwischen dem ersten und zweiten Lagerelement 1*, 2* ist das zu einem vollständigen Ringschluss ergänzte helikalförmige Federelement 5** angeordnet, das lediglich über die quer zur Drehachse D orientierten Federelemente 13, 14, 15, 16, gleichsam wie in Figur 6, mit den bogenförmig ausgebildeten Lagerelementen 1*, 2* monolithisch verbunden ist. Figur 10 b zeigt das Festkörpergelenk ohne die Federelemente 13 bis 16. In dieser Darstellung ist ersichtlich, dass zwischen dem Lagerelement 2* und dem Federelement 5** ein Spalt besteht, ebenso auch zwischen dem Lagerelement 1* und dem Federelement 5**. Ferner verfügen die Lagerelement 1*, 2* über eine größere radiale Erstreckung als das Federelement 5**, so dass ihre über das Federelement 5** radial erhabenen Außenflächen in Wirkverbindung mit zwei um die Drehachse D relativ zueinander dreh- bzw. schwenkbaren Komponentenabschnitte treten können.

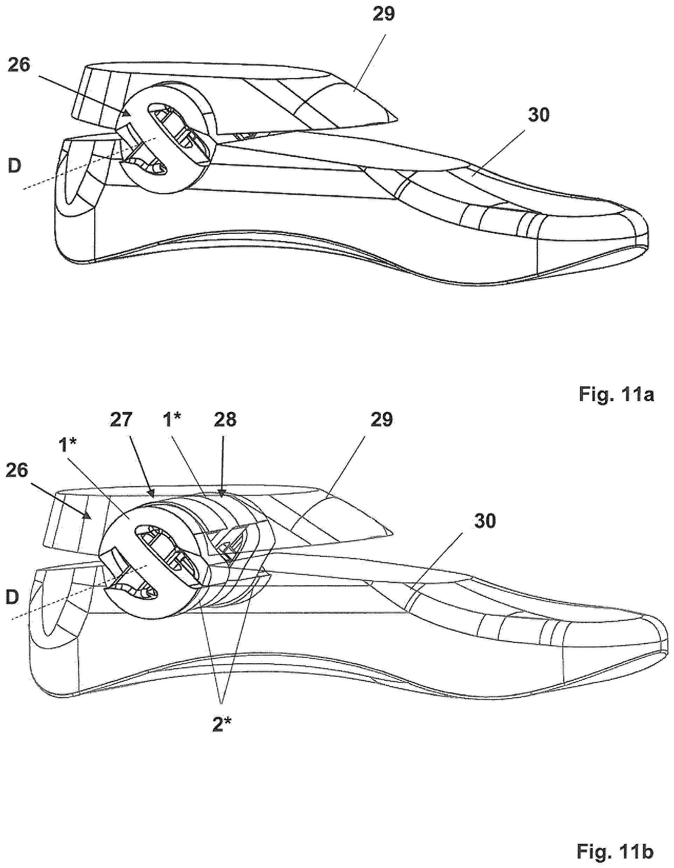

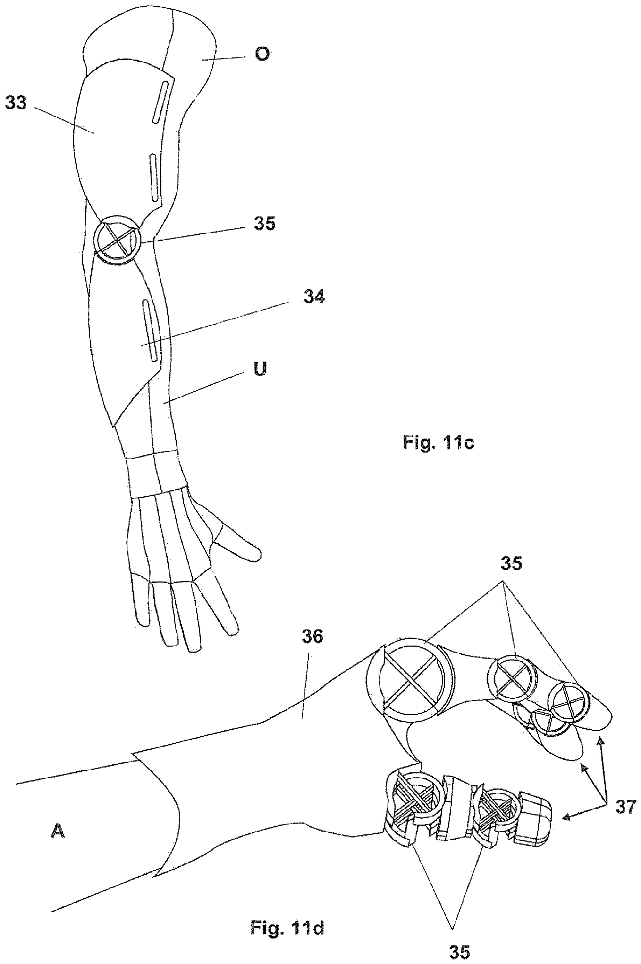

Das in Figur 10 a dargestellte Festkörpergelenk verfügt aufgrund des vollständigen Ringschlusses in der Ausbildung des Federelementes 5** über eine höhere Federfestigkeit sowohl in Bezug auf Drehungen um die Drehachse D als auch in Bezug auf quer zur Drehachse D auf das Federelement einwirkenden Verformungskräfte. Bevorzugte Verwendungen des Festkörpergelenkes sind in den Figuren 11 a, b, c und d gezeigt.

Figuren 11a und b, von denen Figur 11b eine Teiltransparentansicht darstellt, zeigen jeweils eine Fußprothese mit einem Fußgelenk 26, das über zwei parallel geschaltete Festkörpergelenke 27 und 28, jeweils nach Art des in Figur 10 a gezeigten Festkörpergelenkes, verfügt. Das Fußgelenk 26 verbindet einen oberen Prothesenfußteil 29 mit einem unteren Prothesenfußteil 30, die beweglich um die Drehachse D des Fußgelenkes gelagert sind. Hierbei sind die radial erhabenen Aussenflächen der bogenförmig ausgebildeten Lagerelemente mit dem oberen bzw. dem unteren Prothesenfußteil 29, 30 fest verbunden.

Die Figur 11c zeigt eine Ellenbogenorthese, die jeweils eine an den Oberarm O und an den Unterarm U angepasste Orthesenschale 33, 34 aufweist, die beide über ein Festkörpergelenk 35 gelenkig miteinander verbunden sind. Das Festkörpergelenk 35 ist nach einem der vorstehenden Ausführungsbeispiele, vorzugsweise in Art eines in den Figuren 1 bis 4 illustrierten Festkörpergelenkes, ausgebildet. Mit Hilfe der Ellenbogenprothese kann das Ellenbogengelenk unterstützt oder entlastet werden. Auch eine teilweise Einschränkung der Beweglichkeit ist durch spezielle Geometrien oder Sperrungsmechanismen innerhalb des Festkörpergelenkes möglich. Durch eine starke Federung des Festköpergelenkes wäre zudem ein Trainingseffekt möglich. Selbstverständlich lässt sich das der Ellenbogenprothese zugrundeliegende Orthesenprinzip durch entsprechende Form-Abwandlung der Orthesenschalen auf andere Körpergelenke übertragen, z.B. Hüftgelenk, Handgelenk, Kniegelen, Sprunggelenk oder Schulter.

Die Figur 11d zeigt eine Prothesenhand 36, an der gelenkig ausgebildete Fingerelemente 37 angebracht sind. Die Prothesenhand 36 sitzt endseitig an einem Armstrumpf A auf. Längs der Fingerelemente 37 sind zu Zwecken einer Nachbildung der natürlichen Fingerbeweglichkeit Festkörpergelenke 35 unterschiedlicher Größe und Ausprägung nach Art der vorstehend beschriebenen Festkörpergelenke angebracht. Die Festkörpergelenke 35 können optional mit geeigneten Aktoren, bspw. Seilzugaktoren, kombiniert werden, um eine aktive Beugung und Streckung der einzelnen Fingerelemente 37 zu initiieren.

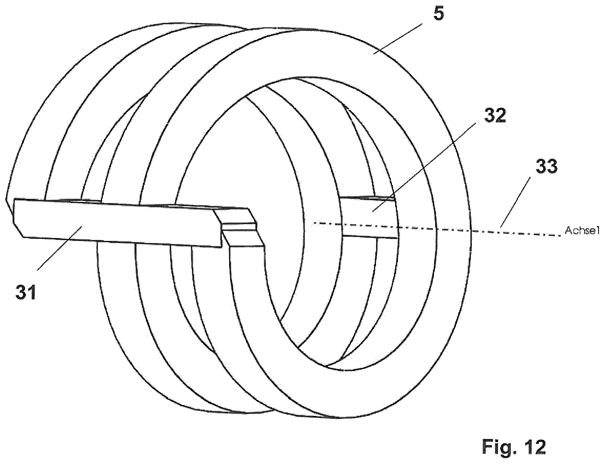

Figur 12 zeigt ein helikalförmiges Federelement 5, das längs zu dessen Steigung zwei benachbarte Federabschnitte unmittelbar miteinander verbindende Verbindungsstege 31, 32 vorsieht. Durch die Verbindungsstege 31, 32, die einzeln oder gemeinsam vorgesehen sein können, erfährt das Federelement eine erhöhte Federsteifigkeit sowohl in Federlängsrichtung als auch in torsionaler Richtung um die Federachse 33. Das dargestellte Federelement kann in allen lösungsgemäß erläuterten Festkörpergelenken eingesetzt werden.

### Bezugszeichenliste

- 1: oberes Lagerelement
- 2: unteres Lagerelement
- 1', 2': Lagerfläche
- 3,4: Abstützelemente
- 3',4': Abstützflächen
- 5, 5*: spiral- oder helikalförmiges Federelement
- 61 bis 68: balkenförmiges Federelement
- 7: Verbindungsmittel
- 8: erster Bogenabschnitt
- 9: zweiter Bogenabschnitt
- 10: trapezförmiger Verbindungsabschnitt
- 11: balkenförmiger Verbindungsabschnitt
- 12: helikaler Verbindungsabschnitt
- 13,14,15,16: Federelement
- 17: Festkörpergelenk
- 18: Festkörpergelenk
- 19: Verbindungsabschnitt
- 20: Festkörpergelenk
- 21: Festkörpergelenk
- 22: Außenverzahnung
- 23: Verzahnungsstruktur
- 24, 25: Struktur
- 26: Fußgelenk
- 27, 28: Festkörpergelenk
- 29: oberes Prothesenfußteil
- 30: unteres Prothesenfußteil
- 31, 32: Verbindungssteg
- 33, 34: Orthesenschale
- 35: Festkörpergelenk
- 36: Prothesenhand
- 37: Fingerelemente
- A: Armstumpf
- O: Oberarm
- U: Unterarm

## Patentansprüche

1. Festkörpergelenk mit zwei schwenkbar um eine Drehachse (D) angeordneten Lagerelementen (1, 2) sowie wenigstens zwei sich quer zur Drehachse (D) erstreckenden Federelementen, die jeweils einseitig mit einem Lagerelement (1) und andererseits mit dem anderen Lagerelement (2) in Wirkverbindung stehen,
**dadurch gekennzeichnet, dass** zwischen beiden Lagerelementen (1,2) ein spiral- oder helikalförmiges Federelement (5, 5*) angeordnet ist, und
dass die wenigstens zwei sich quer zur Drehachse (D) erstreckenden Federelemente (61, 62, 63, 64, 65, 66, 67, 68) jeweils einseitig mit dem spiral- oder helikalförmigen Federelement (5, 5*) und andererseits jeweils mit einem der beiden Lagerelementen (1, 2) verbunden sind.

2. Festkörpergelenk nach Anspruch 1,
**dadurch gekennzeichnet, dass** das spiral- oder helikalförmige Federelement (5, 5*) derart ausgebildet und angeordnet ist, dass eine dem spiral- oder helikalförmigen Federelement (5, 5*) zuordenbare Federachse mit der Drehachse (D) zusammenfällt oder zumindest zu dieser parallel verläuft.

3. Festkörpergelenk nach Anspruch 1 oder 2,
**dadurch gekennzeichnet, dass** das spiral- oder helikalförmige Federelement (5, 5*) ausschließlich über die Federelemente (61, 62, 63, 64, 65, 66, 67, 68) mit den Lagerelementen (1, 2) verbunden und gegenüber den Lagerelementen ansonsten lose gelagert ist.

4. Festkörpergelenk nach einem der Ansprüche 1 bis 3,
**dadurch gekennzeichnet, dass** die Federelemente (61, 62, 63, 64, 65, 66, 67, 68) separat zueinander und jeweils in Form einer zwischen dem spiral- oder helikalförmigen Federelement (5,5*) und jeweils einem Lagerelement (1, 2) zweiseitig endseitig eingespannten Blattfeder ausgebildet sind.

5. Festkörpergelenk nach einem der Ansprüche 1 bis 4,
**dadurch gekennzeichnet, dass** das Festkörpergelenk in Art eines Kreuzfedergelenkes ausgebildet ist, bei dem die sich wenigstens zwei quer zur Drehachse (D) erstreckenden Federelemente (61, 62, 63, 64, 65, 66, 67, 68) kreuzen.

6. Festkörpergelenk nach Anspruch 5,
**dadurch gekennzeichnet, dass** wenigstens zwei sich quer zur Drehachse (D) erstreckenden Federelemente im Bereich der Drehachse (D) lokal verbunden sind.

7. Festkörpergelenk nach einem der Ansprüche 1 bis 6,
**dadurch gekennzeichnet, dass** die jeweils einerseits mit einem Lagerelement (1, 2) sowie andererseits mit dem spiral- oder helikalförmigen Federelement (5, 5*) verbundenen Federelemente (61, 62, 63, 64, 65, 66, 67, 68) in einem nicht ausgelenkten Zustand des Festkörpergelenkes eine Krümmung in Längserstreckung derart aufweisen, dass bei Auslenkung des Festkörpergelenkes die Krümmung reduzierbar ist.

8. Festkörpergelenk nach einem der Ansprüche 1 bis 7,
**dadurch gekennzeichnet, dass** das spiral- oder helikalförmige Federelement (5, 5*) längs eines zwischen beiden Lagerelementen (1, 2) wirkenden Kraftflusses zwischen beiden Lagerelementen (1, 2) angeordnet ist, und
dass das spiral- oder helikalförmige Federelement (5, 5*) zusätzlich zu einer um die Drehachse (D) orientierten federelastischen Eigenverformbarkeit eine längs des zwischen beiden Lagerelementen (1, 2) wirkenden Kraftflusses gerichtete federelastische Eigenverformbarkeit besitzt.

9. Festkörpergelenk nach Anspruch 8,
**dadurch gekennzeichnet, dass** das spiral- oder helikalförmige Federelement (5, 5*) und/oder die Lagerelemente (1, 2) längs des Kraftflusses einander zugewandte Strukturen (22, 23) besitzen, die bei gegenseitiger Annäherung und Ineinandergreifen ein Verschwenken beider Lagerelemente (1, 2) um die Drehachse (D) verhindern.

10. Festkörpergelenk nach Anspruch 8 oder 9,
**dadurch gekennzeichnet, dass** die Strukturen (22, 23) in Art einer Verzahnung ausgebildet sind.

11. Festkörpergelenk nach einem der Ansprüche 1 bis 10,
**dadurch gekennzeichnet, dass** wenigstens zwei Festkörpergelenke (20, 21) vorgesehen sind, von denen jeweils ein Lagerelement (1,2) derart fest mit dem anderen verbunden ist, sodass die Drehachsen (D₂₀, D₂₁) beider Festkörpergelenke (20, 21) koaxial gelagert und beide Festkörpergelenke (20, 21) mechanisch in Reihe geschaltet sind, oder
dass die Drehachsen (D₂₀, D₂₁) beider Festkörpergelenke (20, 21) parallel beabstandet zueinander gelagert und beide Festkörpergelenke (20, 21) mechanisch parallel geschaltet sind.

12. Festkörpergelenk nach einem der Ansprüche 1 bis 11,
**dadurch gekennzeichnet, dass** das Festkörpergelenk einstückig mittels eines generativen Herstellverfahrens hergestellt ist.

13. Verwendung des Festkörpergelenkes nach einem der Ansprüche 1 bis 12 als künstliches Gelenk für Exo- und Endoprothesen sowie in Form eines Orthesengelenkes.

14. Verwendung nach Anspruch 13,
**dadurch gekennzeichnet, dass** die Exoprothese eine Oberarmorthese oder eine Prothesenhand ist.

## Claims

1. A solid-body joint, comprising two bearing elements (1, 2) arranged pivotably about an axis of rotation (D), and at least two spring elements extending transversely to the axis of rotation (D), each of which spring elements are each operatively connected to one bearing element (1) at one end and to the other bearing element (2) at the other end,
**characterized in that** a spiral or helical spring element (5, 5*) is arranged between the two bearing elements (1, 2) and
that the at least two spring elements (61, 62, 63, 64, 65, 66, 67, 68) extending transversely to the axis of rotation (D) are each connected to the spiral or helical spring element (5, 5*) at one end and to one of the two bearing elements (1, 2) at the other end.

2. The solid-body joint according to claim 1,
**characterized in that** the spiral or helical spring element (5, 5*) is constructed and arranged such that a spring axis which is assignable to the spiral or helical spring element (5, 5*) is coincident with the axis of rotation (D) or at least extends parallel thereto.

3. The solid-body joint according to claim 1 or 2,
**characterized in that** the spiral or helical spring element (5, 5*) is connected to the bearing elements (1, 2) solely via the spring elements (61, 62, 63, 64, 65, 66, 67, 68) and is otherwise braced loosely with respect to the bearing elements.

4. The solid-body joint according to any one of claims 1 to 3,
**characterized in that** the spring elements (61, 62, 63, 64, 65, 66, 67, 68) are constructed separately from each other and each is designed in the form of a leaf spring clamped between the spiral or helical spring element (5,5*) and the end of a bearing element (1, 2) at both ends.

5. The solid-body joint according to any one of claims 1 to 4,
**characterized in that** the solid-body joint is constructed in the manner of a cross flexural pivot in which the at least two spring elements (61, 62, 63, 64, 65, 66, 67, 68) extending transversely to the axis of rotation (D) intersect each other.

6. The solid-body joint according to claim 5,
**characterized in that** at least two spring elements extending transversely to the axis of rotation (D) are connected locally in the region of the axis of rotation (D).

7. The solid-body joint according to any one of claims 1 to 6,
**characterized in that** the spring elements (61, 62, 63, 64, 65, 66, 67, 68), each connected at one end to a bearing element (1, 2) and at the other end to the spiral or helical spring element (5, 5*) are curved in the longitudinal extension when the solid-body joint is in an undeflected state in such manner that the curvature can be reduced when the solid-body joint is deflected.

8. The solid-body joint according to any one of claims 1 to 7,
**characterized in that** the spiral or helical spring element (5, 5*) is arranged along a flow of force acting between both bearing elements (1, 2) between both bearing elements (1, 2), and
that besides a resilient self-deformation capability orientated about the axis of rotation (D) the spiral or helical spring element (5, 5*) also has a resilient self-deformation capability directed along the flow of force acting between the two bearing elements (1, 2).

9. The solid-body joint according to claim 8,
**characterized in that** the spiral or helical spring element (5, 5*) and/or the bearing elements (1, 2) have structures (22, 23) facing each other along the flow of force which when approaching and engaging with each other prevent both bearing elements (1, 2) from pivoting about the axis of rotation (D).

10. The solid-body joint according to claim 8 or 9,
**characterized in that** the structures (22, 23) are formed in the manner of a toothing.

11. The solid-body joint according to any one of claims 1 to 10,
**characterized in that** at least two solid-body joints (20, 21) are provided, one bearing element (1, 2) of each of which is firmly connected to the other in such manner that the axes of rotation (D₂₀, D₂₁) of the two solid-body joints (20, 21) are mounted coaxially and both solid-body joints (20, 21) are mechanically connected in series, or
that the axes of rotation (D₂₀, D₂₁) of the two solid-body joints (20, 21) the two solid-body joints (20, 21) are mounted at a distance from each other and mechanically connected in parallel.

12. The solid-body joint according to any one of claims 1 to 11,
**characterized in that** the solid-body joint is produced as a single part by means of a generative manufacturing method.

13. Use of the solid-body joint according to any one of claims 1 to 12 as an artificial joint for exo- and endoprostheses and in the form of an orthotic joint.

14. Use according to claim 13,
**characterized in that** the exoprosthesis is an upper arm orthosis or a prosthetic hand.

## Revendications

1. Articulation à corps solide avec deux éléments de palier (1, 2) disposés de manière pivotante autour d'un axe de rotation (D) ainsi qu'au moins deux éléments de ressort s'étendant transversalement à l'axe de rotation (D), qui sont respectivement en liaison opérationnelle d'un côté avec un élément de palier (1) et d'un autre côté avec l'autre élément de palier (2),
**caractérisé en ce que** entre les deux éléments de palier (1, 2) un élément de ressort (5, 5*) en forme de spirale ou hélicoïdal est disposé et
**en ce que** les au moins deux éléments de ressort (61, 62, 63, 64, 65, 66, 67, 68) s'étendant transversalement à l'axe de rotation (D) sont respectivement reliés d'un côté avec l'élément de ressort en forme de spirale ou hélicoïdal (5, 5*) et d'un autre côté avec un des deux éléments de palier (1, 2) .

2. Articulation à corps solide selon la revendication 1,
**caractérisée en ce que** l'élément de ressort en forme de spirale ou hélicoïdal (5, 5*) est configuré et disposé de telle sorte que un axe de ressort pouvant être coordonnée à l'élément de ressort en forme de spirale ou hélicoïdal (5, 5*) coïncide avec l'axe de rotation (D) ou s'étende au moins parallèlement à celui-ci.

3. Articulation à corps solide selon la revendication 1 ou 2,
**caractérisée en ce que** l'élément de ressort en forme de spirale ou hélicoïdal (5, 5*) est relié exclusivement par l'intermédiaire des éléments de ressort (61, 62, 63, 64, 65, 66, 67, 68) avec les éléments de palier (1, 2) et positionné sinon de manière relâchée par rapport aux éléments de ressort.

4. Articulation à corps solide selon une des revendications 1 à 3,
**caractérisée en ce que** les éléments de ressort (61, 62, 63, 64, 65, 66, 67, 68) sont configurés séparément les uns des autres et respectivement sous la forme d'un ressort à lames encastré des deux côtés aux extrémités entre l'élément de ressort en forme de spirale ou hélicoïdale (5, 5*) et un élément de palier respectif (1, 2).

5. Articulation à corps solide selon une des revendications 1 à 4,
**caractérisée en ce que** l'articulation à corps solide est configurée à la manière d'une articulation à ressort croisé, dans laquelle au moins deux éléments de ressort (61, 62, 63, 64, 65, 66, 6, 76, 8) s'étendant transversalement à l'axe de rotation (D) se croisent.

6. Articulation à corps solide selon la revendication 5,
**caractérisée en ce que** au moins deux éléments de ressort s'étendant transversalement à l'axe de rotation (D) sont reliés localement au niveau de l'axe de rotation (D).

7. Articulation à corps solide selon une des revendications 1 à 6,
**caractérisée en ce que** les éléments de ressort (61, 62, 63, 64, 65, 66, 67, 68) reliés respectivement d'un côté avec un élément de palier (1, 2) et d'un autre côté avec l'élément de ressort en forme de spirale ou hélicoïdale (5, 5*) présentent dans un état non dévié de l'articulation à corps solide une incurvation en extension longitudinale, de sorte que lors de la déviation de l'articulation à corps solide l'incurvation puisse être réduite.

8. Articulation à corps solide selon une des revendications 1 à 7,
**caractérisée en ce que** l'élément de ressort en forme de spirale ou hélicoïdal (5, 5*) est disposé le long d'un flux de force agissante entre les deux éléments de palier (1, 2) entre les deux éléments de palier (1, 2), et
**en ce que** l'élément de ressort en spirale ou hélicoïdal (5, 5*) possède en plus d'une déformabilité propre élastique orientée autour de l'axe de rotation (D) une déformabilité propre élastique alignée le long du flux de force agissant entre les deux éléments de palier (1, 2).

9. Articulation à corps solide selon la revendication 8,
**caractérisé en ce que** l'élément de ressort en forme de spirale ou hélicoïdal (5, 5*) et/ou les éléments de palier (1, 2) possèdent le long du flux de force des structures tournées l'une vers l'autre (22, 23), qui en cas de proximité mutuelle empêchent une mise en prise l'un dans l'autre et un basculement des deux éléments de palier (1, 2) autour de l'axe de rotation (D) .

10. Articulation à corps solide selon la revendication 8 ou 9,
**caractérisée en ce que** les structures (22, 23) sont configurées à la manière d'une denture.

11. Articulation à corps solide selon une des revendications 1 à 10,
**caractérisée en ce que** au moins deux articulations à corps solide (20, 21) sont prévues, desquelles respectivement un élément de palier (1, 2) est solidement relié avec l'autre de telle sorte que les axes de rotation (D₂₀, D₂₁) des deux articulations à corps solide (20, 21) soient positionnées coaxialement et les deux articulations à corps solide (20, 21) soient branchées mécaniquement en série ou
**que** les axes de rotation (D₂₀, D₂₁) des deux articulations à corps solide (20, 21) soient positionnées en étant espacées les unes des autres et les deux articulations à corps solide (20, 21) soient branchées mécaniquement en parallèle.

12. Articulation à corps solide selon une des revendications 1 à 11,
**caractérisée en ce que** l'articulation à corps solide est fabriquée en un seul tenant au moyen d'un procédé de fabrication génératif.

13. Utilisation de l'articulation accord solide selon une des revendications 1 à 12 comme articulation artificielle pour des exoprothèses et endoprothèses ainsi que sous la forme d'une orthèse d'articulation.

14. Utilisation selon la revendication 13,
**caractérisée en ce que** l'exopiothèse est une orthèse du haut du bras ou une prothèse de main.
